Europäisches Patentamt

European Patent Office

Office européen des brevets

(11) Publication number: **0 190 718**
**B1**

# EUROPEAN PATENT SPECIFICATION

(45) Date of publication of patent specification: **24.05.89**

(51) Int. Cl.⁴: **G 01 N 1/22**

(21) Application number: **86101418.1**

(22) Date of filing: **04.02.86**

(54) **Sampling device for gas analyzers.**

(30) Priority: **07.02.85 JP 16787/85 u**

(43) Date of publication of application:
**13.08.86 Bulletin 86/33**

(45) Publication of the grant of the patent:
**24.05.89 Bulletin 89/21**

(84) Designated Contracting States:
**DE FR GB**

(56) References cited:
**FR-A-2 107 237**
**US-A-3 748 906**

**JOURNAL OF CHROMATOGRAPHIC SCIENCE,**
**vol. 9, no. 8, August 1971, pages 492-496, Niles,**
**US; N.L. SOULAGES et al.: "The analysis of**
**hydrocarbon types-saturates, aromatics and**
**olefins-by selective chemical absorption and**
**flame ionization detection"**

(73) Proprietor: **HORIBA, LTD.**
**2 Miyanohigashi-machi Kissyoin**
**Minami-ku Kyoto (JP)**

(72) Inventor: **Noguchi, Naoki**
**935-44, Kojima-cho**
**Moriyama-city Shiga-prefeCture (JP)**
Inventor: **Ohnishi, Toshikazu**
**495-8, Heso Ritto-cho**
**Kurita-gun Shiga-prefecture (JP)**

(74) Representative: **Patentanwälte TER MEER -**
**MÜLLER - STEINMEISTER**
**Mauerkircherstrasse 45**
**D-8000 München 80 (DE)**

Courier Press, Leamington Spa, England.

## Description

The invention relates to a sampling device for gas analyzers using gas detectors such as a FID (Flame Ionization Detector) and a CLD (Chemical Luminescence Detector).

Since the quantity of a sample introduced into gas detectors such as a FID and a CLD is small, e.g. several cm³/min to several 10 cm³/min, the inside diameter of a capillary used in the control of the flow rate must be remarkably small, i.e. in the range of 0.1 to 0.3 mm with the inherent defect that the capillary is inclined to be choked.

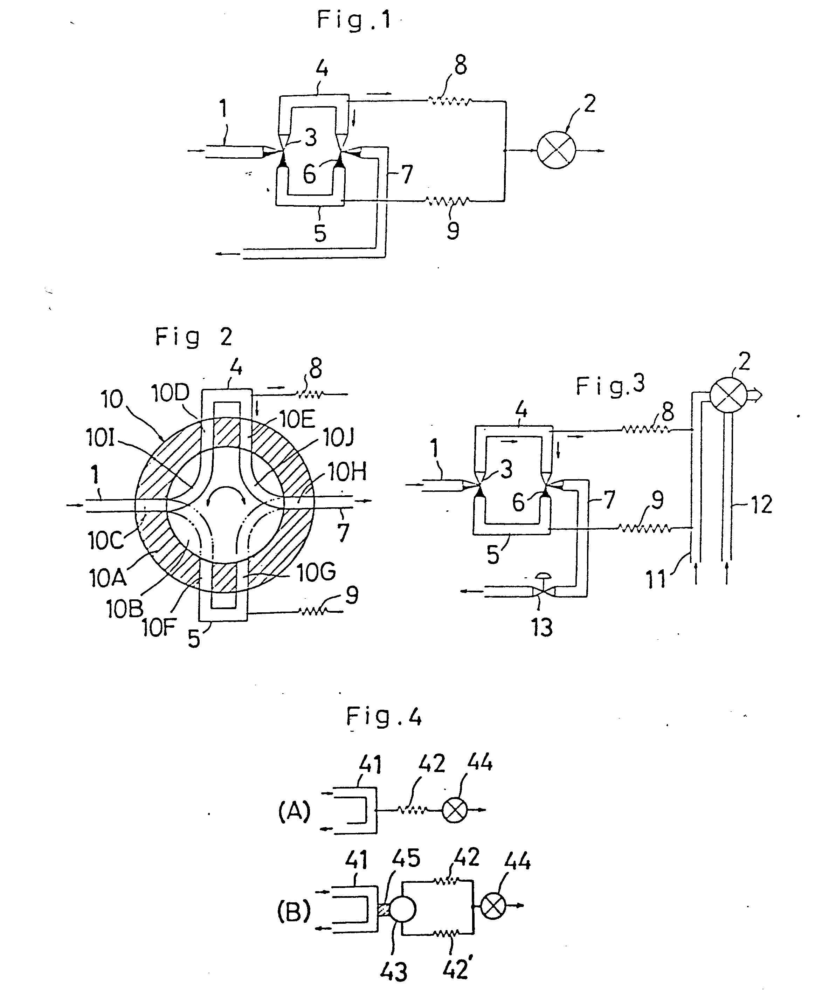

Sampling devices are known in which a sample-supply passage 41 is provided with one capillary 42 connected thereto, as shown in Fig. 4(A), or in which two capillaries 42, 42' are connected in parallel and selectively changed-over by means of a three-way change-over valve 43, as shown in Fig. 4(B). Reference numeral 44 designates a detector.

With the conventional sampling device shown in Fig. 4(A), in the event that the capillary 42 is choked it must be renewed to continue the measuring operation. It costs much time to replace the capillary and it takes several hours to wait for the stabilization of a desired temperature and the fall of a background after the replacement of the capillary. As a result, if the capillary is choked during the measurement, a great problem may arise due to the fact that owing to the repair the measurement can not be carried out for a long time. One particular example is the so called on line measurement such as the checking of the exhaust gas components from cars in production process, during later checking in a testing station and so on.

Also, with the conventional sampling device shown in Fig. 4(B), in which the capillaries 42, 42' are connected in parallel, a passage 45 (hatched portion) and the valve 43 used for changing-over have too large a volume in comparison with the flow rate of a sample sent to the detector 44 through the capillaries 42, 42', thus forming a dead space, resulting in the defect that it takes a long time to replace the gas and the response speed of the gas analyzer is remarkably reduced.

The present invention was achieved with an eye on the above described defects and aims at the provision of a sampling device for an analyzer which is capable of changing over the small flow rate capillaries without inducing a response delay to the detector.

The above described object is achieved by the device according to claim 1.

A six-way valve in which the functions of two three-way valves are substantially brought together, as shown in Fig. 2, can be used for said first changing-over valve and said second changing-over valve in addition to a three-way changing-over valve as shown in Fig. 1. Also, these valves may be operated either manually or automatically.

In addition, in order to apply to a FID, it is necessary only to connect a downstream side of each capillary to a fuel-supply passage connected to a detector. Also, a by-pass passage may be provided with a back pressure regulator.

With the above described construction, in the event that one capillary is choked, the first changing-over valve and the second changing-over valve are operated, respectively, to stop an inflow of a sample to one capillary side and supply a detector side with a sample through another capillary. In addition, since only a required quantity of sample gas is introduced into a capillary from a branched passage and the rest is flown fast to a by-pass side, the replacement time of gas can be shortened with the advantage of preventing a delay of the response speed of the detector.

Embodiments of the invention will be described in the following with reference to the accompanying drawings.

Fig. 1 is a block diagram showing the principal parts in one embodiment of the present invention;

Fig. 2 is a block diagram showing the principal parts of still another embodiment of the invention; and

Fig. 3 is a block diagram showing the principal parts of still another embodiment of the invention; and

Figs. 4(A), (B) are diagrams showing the conventional sample-supply passage arrangements in gas analyzers.

Referring now to Fig. 1, a sample-supply passage 1 is connected with its upstream side to a sample gas source (not shown). A first changing-over valve 3 such as a three way changing-over valve is disposed on the downstream side of said sample-supply passage 1. A first branched passage 4 and a second branched passage 5 are connected in parallel to each other on the downstream side of said first changing-over valve 3. A second changing-over valve 6 which is a three-way changing-over valve similarly to said first changing-over valve 3 is connected at a joint of said both branched passages 4, 5, followed on the downstream side by a by-pass passage 7. Capillaries 8, 9 (hereinafter referred to as a first capillary 8 and a second capillary 9, respectively) are connected midway of said first branched passage 4 and said second branched passage 5, respectively, the downstream sides of said both capillaries 8, 9 being connected to a detector 2.

In operation of a sampling device having the above described construction in the event that both the first changing-over valve 3 and the second changing-over valve 6 are opened on the first branched passage 4 side (the condition shown in Fig. 1), a sample sent through the sample-supply passage 1 reaches the detector 2 through the first capillary 8 and the rest of the sample is fast exhausted through the second changing-over valve 6 and the by-pass passage 7.

Under this condition, if said capillary 8 is choked, upon operating the first changing-over valve 3 and the second changing-over valve 6 so as to open the second branched passage 5 side, the sample is supplied to the detector 2 through the second capillary 9. Since, at this time, a sample remaining within the first changing-over valve 3 and the second branched passage 5 is fast flown into the by-pass passage 7 through the second branched passage 5 and the second changing-over valve 6 having a small flow-resistance. The detector 2 is supplied with a fresh sample through the second capillary 9 without delay and after that, the rest of the sample gas flowing through the second capillary 9 is fast exhausted through the second changing-over valve 6 and the by-pass passage 7. The first choked capillary 8 is restored to the former condition by repairing, replacing and the like without any noteworthy interruption of the measuring efficiency of the gas analyzer.

Fig. 2 shows another embodiment wherein the functions of two changing-over valves 3, 6 used in the above described embodiment are achieved by one valve, that is to say a six-way valve 10 with a construction as described in the following. Reference numeral 10A designates a fixed wall,

— 10B designates a rotable valve body,

— 10C designates a connecting port for the sample-supply passage 1,

— 10D, 10E designate connecting ports to which the first branched passage 4 is connected,

—10F, 10G designate connecting ports to which the second branched passage 5 is connected,

— 10H designates a connecting port to which the by-pass passage 7 is connected, and

— 10I, 10J designate a connecting passage formed independently to each other within said valve body 10B, respectively.

The connecting ports 10C, 10D are arranged in a 180°-symmetrical relation to the connecting ports 10H, 10G, the connecting ports 10E, 10H to the connecting ports 10F, 10C, and the connecting port 10C to the connecting port 10H. Accordingly, either of the first branched passage 4 and the second branched passage 5 can be connected to the sample-supply passage 1 by rotating the valve body 10B in Fig. 2 by 180° such that the detector 2 can be supplied with a sample through either the first capillary 8 or the second capillary 9.

Fig. 3 shows still another embodiment wherein the detector 2 is a FID. A fuel-supply pipe 11 is connected to the downstream sides of the first capillary 8 and the second capillary 9. An air-supply pipe 12 serves for supplying the detector 2 with air. A back pressure regulator is provided in the by-pass passage 7.

As explained by the above detailed description of the embodiments, according to the present invention, in the event that a capillary is choked, a valve is changed over to exhaust an accumulation within the valve without delay whereby the measurement can be continued without causing a delay in the response at the beginning. Also, since the gas except a gas introduced into the capillary is exhausted through a by-pass line without delay even when an analyzer is ready for operation and since the capillary is supplied with a required quantity of a fresh sample gas in turn, substantially no response delay has to be taken into account any more. The choked capillary can be replaced timely and repair during a tie-up of operation of a production line, measuring equipment and the like is easily possible whereby the reduction of running time of the line can be reduced. Also, the degree of choking in the capillary can be checked by changing over it sometimes whereby the need of replacement and the replacing time of a capillary can be estimated. With the present invention, a dead space as shown by reference numeral 45 in Fig. 4(B), and a delay in response resulting from it are avoided and a more accurate measurement can be achieved.

## Claims

1. A sampling device for a gas analyzer, comprising

a detector (2) connected to the downstream sides of two capillaries (8, 9),

— a first branched passage (4) and a second branched passage (5) being connected at their upstream ends to a sample-supply passage (1) through a first changing-over valve (3),

— a second changing-over valve (6) being connected to a by-pass passage (7) at the downstream joint of said two branched passages (4, 5), and

— said first branched passage (4) and said second branched passage (5) being connected each to one of said two capillaries (8, 9), respectively at the upstream sides of said capillaries.

2. The sampling device as set forth in Claim 1, characterized in that said first changing-over valve (3) and said second changing-over valve (6) are three-way changing-over valves, respectively.

3. The sampling device as set forth in Claim 1, characterized in that said first changing-over valve (3) and said second changing-over valve (6) are integrated forming one multiple passage valve.

4. The sampling device as set forth in Claim 2, characterized in that said first three-way changing-over valve (3) and said second three-way changing-over valve (6) are integrated forming one six-way valve body (10).

## Patentansprüche

1. Probenentnahmevorrichtung für einen Gasanalysator, mit

— einem Detektor (2), der mit den stromabwärts befindlichen Seiten von zwei Kapillaren (8, 9) verbunden ist,

— einem abgezweigten ersten (4) und einem abgezweigten zweiten Durchlaß (5), deren stromaufwärts befindliche Enden über ein erstes Umschaltventil (3) mit einem Probenzuführungs-Durchlaß (1) verbunden sind,

— einem zweiten Umschaltventil (6), das an der stromabwärts befindlichen Verbindungsstelle der beiden abgezweigten Durchlässe (4, 5) an einen Umgehungsdurchlaß (7) angeschlossen ist,

wobei der erste abgezweigte Durchlaß (4) bzw.

der zweiten abgezweigte Durchlaß (5) jeweils mit einem stromaufwärts befindlichen Ende der beiden Kapillaren (8, 9) verbunden ist.

2. Probenentnahmevorrichtung nach Anspruch 1, dadurch gekennzeichnet, daß das erste (3) bzw, das zweite Umschaltventil (6) jeweils aus einem Drei-Wege-Ventil besteht.

3. Probenentnahmevorrichtung nach Anspruch 1, dadurch gekennzeichnet, daß das erste (3) und das zweite Umschaltventil (6) als Einheit ausgebildet sind und ein einziges Mehrwegeventil bilden.

4. Probenentnahmevorrichtung nach Anspruch 2, dadurch gekennzeichnet, daß das erste (3) und das zweite Drei-Wege-Ventil (6) als Einheit ein einziges Sechs-Wege-Ventil (10) bilden.

**Revendications**

1. Dispositif d'échantillonnage pour un analyseur de gaz, comportant
   — un détecteur (2) relié aux côtés aval de deux capillaires (8, 9)
   — un premier passage ramifié (4) et un second passage ramifié (5) reliés à leurs extrémités amont à un passage (1) de délivrance d'échantillon par l'intermédiaire d'une première vanne de commutation (3),
   — une seconde vanne de commutation (6) étant reliée à un passage de dérivation (7) à la jonction aval desdits deux passages ramifiés (4, 5), et
   .— Ledit premier passage ramifié (4) et ledit second passage ramifié (5) étant reliés chacun à l'un desdits deux capillaires (8, 9), respectivement, sur les côtés amont desdits capillaires.

2. Dispositif d'échantillonnage selon la revendication 1, caractérisé en ce que ladite première vanne de commutation (3) et ladite seconde vanne de commutation (6) sont des vannes de commutation à trois voies, respectivement.

3. Dispositif d'échantillonnage selon la revendication 1, caractérisé en ce que ladite première vanne de commutation (3) et ladite seconde vanne de commutation (6) sont intégrées constituant une vanne à passages multiples.

4. Dispositif d'échantillonnage selon la revendication 2, caractérisé en ce que ladite première vanne de commutation à trois voies (3) et ladite seconde vanne de commutation à trois voies (6) sont intégrées constituant un corps de vanne à six voies (10).

Fig.1

Fig 2

Fig.3

Fig.4

(A)

(B)